# EUROPEAN PATENT SPECIFICATION

(11) **EP 1 131 104 B1**
(45) Date of publication and mention of the grant of the patent: **02.02.2005**
(21) Application number: 99972109.5
(22) Date of filing: 12.11.1999
(51) Int. Cl.: A61K 47/44, A61K 47/14, A61P 31/22

(54) **ANTIVIRAL FORMULATIONS COMPRISING PROPYLENE GLYCOL AND AN ISOPROPYL ALKANOIC ACID ESTER**
ANTIVIRALE ZUSAMMENSETZUNGEN WELCHE PROPYLENGLYKOL UND ISOPROPYL-ALKANSÄUREESTER ENTHALTEN
FORMULATIONS ANTIVIRALES RENFERMANT DU PROPYLENE GLYCOL ET UN ESTER ACIDE ALKANOIQUE D'ISOPROPYLE

(30) Priority: 18.11.1998 SE 9803929
(43) Date of publication of application: 12.09.2001
(73) Proprietor: MEDIVIR AB, S-144 44 Huddinge (SE)
(72) Inventor: LEKARE, Gunilla, S-784 50 Borlänge (SE)
(74) Representative: Dehmel, Albrecht, Dr.
(86) International application number: PCT/SE1999/002043
(87) International publication number: WO 2000/029027

(56) References cited:
- WO-A1-96/24355
- WO-A1-98/17316

## Description

### Technical Field

This invention relates to topical antiviral formulations suitable for orofacial or genital application and comprising an antiinflammatory agent and an antiviral agent. The invention further relates to the treatment or prophylaxis of herpes virus diseases using such formulations.

### Background Art

International patent application no WO 96/24355 describes pharmaceutical compositions comprising a topically acceptable antiviral substance and an antiinflammatory glucocorticoid in a pharmaceutically acceptable carrier. The carriers disclosed in this application comprise conventional formulations for each of the respective active agents, that is a conventional antiviral formulation is combined with a conventional glucocorticoid formulation. Such combined formulations have a large number of ingredients, with potential for interreacting with each other, leading to unknown stability and physicochemical characteristics.

European patent application no. EP 44543 relates to oil in water formulations of the acyclic nucleoside antiviral agent acyclovir and describes that effective topical penetration necessitates that the carrier comprises at least 30 weight percent, preferably at least 40 weight percent propylene glycol. This formulation, denoted the MAC formulation, forms the basis of the most widely marketed topical acyclovir preparation.

International patent application no WO 91/11187 relates to oil in water or aqueous topical formulations of the acyclic nucleoside antiviral agent penciclovir. These formulations must comprise at least 30 weight percent, preferably at least 35 weight percent, propylene glycol. European patent application no. EP 416 739 relates to topical formulations of penciclovir comprising at least 30 weight percent propylene glycol and a decyl methyl sulfoxide emulsifier. International patent application no. WO 93/00905 relates to topical formulations of penciclovir comprising at least 30 weight percent, preferably at least 35 weight percent, propylene glycol and a cetomacrogol 1000 emulsifier.

Formulation of a combination product comprising a strongly lipophilic component such as hydrocortisone and a moderately lipophobic component such as an acyclic nucleoside analogue is difficult in the case of conventional pharmaceuticals, but is even more difficult in the case of a topical preparation where release rates of the active ingredients into the target tissue must be taken into account. As described in the publications in the two paragraphs immediately above, it has hitherto been regarded essential to utilise a high level of propylene glycol when formulating acyclic nucleosides in order to achieve adequate penetration of the antiviral agent into the skin or dermal mucosa. We have discovered, however, that while conventional, high concentration propylene glycol carriers can be used to prepare efficacious antiviral compositions within the scope of the above mentioned WO 96/24355 (that is compositions comprising a glucocorticoid and an antiviral agent), these conventional carriers result in the product having an undesirably short shelf life and less than optimal antiviral and therapeutic performance.

### Disclosure of the Invention

In accordance with the invention there is provided a topical composition as defined by claim 1.

In the context of the invention, this combination of a lower concentration of propylene glycol in conjunction with an isopropyl alkanoic acid ester allows for good penetration and release of the antiviral component, while at the same time avoiding the instability shown by conventional antiviral compositions.

The compositions of the invention are useful for the treatment or prophylaxis of diseases caused by members of the herpes virus family, such as herpes simplex type 1 (predominantly an orofacial infection), herpes simplex type 2 (predominantly a genitoanal infection), varicella zoster virus primary infection (chicken pox) and secondary infection (shingles), human herpes virus type 6 and 8 (implicated in the skin condition Kaposi's sarcoma) and the like. Prophylaxis in the context of the invention includes prevention of infection (including preventing spread to adjacent healthy tissue) and preventing reactivation of previous herpes virus infection, such as reactivation of herpes lying dormant in neural tissue.

A further aspect of the invention thus provides the use of the composition defined above in medicine, particularly in the manufacture of a topical medicament for the treatment or prophylaxis of herpes virus infections in humans, especially herpes simplex type 1 and herpes simplex type 2.

Weight percentages herein refer to the weight of the component relative to the weight of the composition.

Particularly preferred antiviral agents include penciclovir, 9-[4-hydroxy-2-(hydroxymethyl)butyl]guanine (H2G) and especially acyclovir. The antiviral agent may be in the form of a prodrug which is metabolised *in situ,* for instances with epidermal esterases or xanthine oxidases, to form an antiviral agent such as the ones listed above.

The antiviral agent is included in the formulation in substantially conventional concentrations for the respective nucleoside: preferably 1-7 weight percent such as around 4-5 weight per cent. Advantageously the formulation is largely or completely saturated with respect to the antiviral agent.

Hydrocortisone and its esters are particularly preferred.

The hydrocortisone (glucocorticoid) is included in the formulation in substantially conventional concentrations for the respective glucocorticoid, which concentrations are well known in the glucocorticoid art and generally fall in the range of 0.1 to 10 weight percent. For instance, the formulation may contain 0.5 to 2 weight per cent, such as about 1 weight per cent hydrocortisone.

The glucocorticoid and antiviral components may be in substantially dissolved form, dependent upon the carrier, but are conveniently prepared from a micronised raw material, such as those having > 75 %, preferably greater than 90% of particles with less than a defined particle size. For instance the glucocorticoid hydrocortisone is conveniently prepared from a raw material with a particle size less than 5 µm. The antiviral acyclovir or penciclovir is conveniently presented with a particle size less than 15 µm, preferably less than 7 µm.

The compositions of the invention are biphasic and comprise discrete oil and aqueous phases, that is an oil in water emulsion. Preferably the composition comprises a dispersed oil phase and a continuous aqueous phase. The isopropyl alkanoic acid ester and the typically lipophilic glucocorticoid component will be preferentially found in the oil phase, while the antiviral nucleoside will generally be found in the aqueous phase, typically in conjunction with the propylene glycol.

Components of the oil phase may include conventional fats and oils and their esters, as found in the European and other pharmacopoeias. Oil phase components are preferably non-greasy, non staining and washable. Conventional pharmaceutical oil phase components include mineral oils such as vaseline, liquid paraffin and the like, alkanoic acids such as stearic acid and fatty alcohols such as cetostearyl alcohol, straight or branched chain mono or dibasic alkyl esters such as di-isopropyl adipate, isocetyl stearate, propylene glycol diester of coconut fatty acids, decyl oleate, butyl stearate, 2-ethylhexyl palmitate and other 2-ethylhexanoic acid esters and the like.

Preferred isopropyl alkanoic acid esters include the dodecanate, myristate, palmitate, stearate, eicosanate or behenoate esters, especially isopropyl myristate. The composition of the invention comprises about 10 to about 25 weight per cent of the isopropyl alkanoic acid ester, preferably about 12 to about 18 weight per cent, such as about 15 weight per cent.

The composition of the invention comprises about 15 to about 25 weight percent propylene glycol, such as around 18 to around 22 weight per cent. Conveniently the propylene glycol content is around 20 weight per cent as this concentration generally assures a good preservative effect without needing exogenous preservatives in the composition.

The composition of the invention conveniently includes an emulsifier (surfactant), typically in an amount of 0.05 to 5, preferably 0.1 to 1 weight per cent. The European Pharmacopoeia describes a number of pharmaceutically acceptable emulsifiers including anionic, cationic and non-ionic emulsifiers.

Exemplary non-ionic emulsifiers include cetomacrogols, such as cetomacrogol 1000, ethylene or diethylene glycol monostearate, glyceryl esters such as the behenate, oleate, stearate etc, laureth compounds such as lauromacrogols, macrogol monomethyl ethers, mono- and diglycerides, nonoxinols, octoxinols, poloxamers such as poloxamer 407, polyoxyl castor oils, polyoxyl stearates, polysorbates, polyvinyl alcohols, propylene glycol diacetates, sorbitan esters and the like. Poloxamer 188 is a preferred non-ionic surfactant.

Exemplary anionic emulsifiers include aluminium monostearate, calcium stearate, sulphated castor oil, magnesium stearate, pendecamaine, sodium oleate, sodium stearate, sodium stearyl fumarate, sodium tetradecyl sulphate, zinc stearate and the like. A preferred anionic emulsifier is sodium lauryl sulphate

The compositions of the invention can also include conventional auxiliaries such as surface anaesthetics, sunscreens, flavours, scents, emollients or skin tone colourants and masks.

The compositions of the invention can be prepared by conventional blending techniques. Preferably the compositions are prepared by conventional biphasic blending techniques, whereby the oil and aqueous/propylene glycol phases are separately blended and homogenised and brought to a common temperature before mixing. The active ingredients (that is the glucocorticoid and the nucleoside analogue) may be added to their respective oil and aqueous phases before or after blending. Preferably, to minimize the tendency to recrystallisation, the active ingredients are added after blending of the two phases. This means that there is a greater volume when the active ingredients are added, and additionally the biphasic mixture is generally at a lower temperature.

The intended viral conditions, such as herpes simplex lesions on the lips and/or genitalia or herpes zoster (shingles), are episodic. As with all antiviral treatments it is desirable to commence application of the medicament as soch as possible after the reactivation of a dormant herpes infection into an incipient lesion is sensed or suspected. For instance many people experience a warmth or tingling at the coming focal point one or more days before the first visual signs of a herpes lesion become discernable. Application of the composition of the invention is preferably commenced at this point. In some patients, exposure to certain stimuli, such as UV light when skiing or from tropical sun, severe emotional stress or menstruation, can induce reactivation of herpes lesions in particular positions. The composition of the invention can be applied in a prophylactic manner upon exposure to these stimuli. In either event it will be convenient for people prone to herpes lesions to keep a supply of the composition readily available for speedy application when needed. Accordingly it is desirable for the composition of the invention to have a long shelf life without refrigeration, so that the medicament can be kept at home or at work and/or packed for travel.

The composition will generally be applied to the incipient or apparent lesion two to twelve times per day during an episode, such as every three hours. Application preferably continues at least until the hard scab stage which generally takes 3 to 10 days from the first sensation that an episode is expected.

The composition of the invention is preferably presented in a tube containing 0.25 to 50 ml. Conveniently the tube contains sufficient for a single cold or genital sore episode, such as 1 to 5 ml. This will allow several daily applications over no more than a week or ten days, the residue being discarded, thus minimizing potential contamination of the open tube and/or cross infection between individuals sharing the same tube.

Preclinical efficacy of compositions of the invention can be assayed as shown in the examples or with the adoptive transfer of immunity model described in WO 96/24355 and WO 96/24963

### Brief Description of the Drawings

Various embodiments of the invention will now be illustrated by way of example only with reference to the following non-limiting examples and drawings in which:
Fig 1 is a plot of lesion score against time for herpes infected guinea pigs topically receiving a composition of the invention or a placebo;
Fig 2 is a plot of lesion score against time for herpes infected guinea pigs topically receiving a composition comprising a conventional carrier or a placebo;
Fig 3 is plot of lesion score against time for herpes infected guinea pigs topically'receiving a composition falling outside the scope of the invention or a placebo;
Fig 4 is a micrograph showing crystal growth in a conventional formulation;
Fig 5 is a micrograph showing the lack of crystal growth in a formulation of the invention;
Fig 6 is a plot of lesion score against time for herpes infected guinea pigs topically receiving a composition of the invention or a composition prepared according to WO 96/24355, example 3;
Fig. 7 is a plot of lesion score against time for herpes infected guinea pigs topically receiving an alternative composition of the invention or a composition prepared analogously to WO96/24355.

### Detailed Description

### Example 1

A composition in accordance with the invention is prepared from the following ingredients:

| oil phase | | |
|---|---|---|
| cetostearyl alcohol | 6.75 g | 6:75% |
| vaseline | 10.00 g | 10.0% |
| liquid paraffin | 5.65 g | 5.65% |
| isopropyl myristate | 15.00 g | 15.0% |

| aqueous phase | | |
|---|---|---|
| propylene glycol | 20.00 g | 20.0% |
| sodium lauryl sulphate | 0.80 g | 0.8% |
| poloxamer 188 | 1.00 g | 1.0% |
| aq. purif. | 34.8 g | 34.8% |

| active components | | |
|---|---|---|
| acyclovir | 5.00 g | 5.0% |
| hydrocortisone | 1.00 g | 1.0% |

The particle size of the acyclovir (Recordati micronised, USP23/BP93/Eur Ph III) was 10% = 2 µm, 50% = 4 µm, 90% = 7 µm & 100% = 15 µm. The particle size of the hydrocortisone (Pharmacia & Upjohn micronised USP/EP/BP) was 100% <5 µm, geometric mean diameter 2 µm. The purified water is reverse osmosis treated.

The oil phase and aqueous phase components are added to respective mixing vessels, which are each heated to 70°C under agitation. When the phases are at an identical temperature, the oil phase is poured onto the aqueous phase from above while continuing to agitate for 3-5 minutes at the highest possible speed which avoids drawing air into the mixture. The thus emulsified mixture is then homogenised and cooled, with continued agitation, to 32-25°C. The active ingredients are added and agitation continued until the active ingredients are wetted and blended in. The mixture is once again homogenised and cooled until the cream thickens, around 30°C, before packaging.

### Example 2

A penciclovir/hydrocortisone composition according to the invention was prepared from the following components:

| oil phase | | |
|---|---|---|
| cetostearyl alcohol | 6.75 g | 6.75% |
| vaseline | 10.00 g | 10.0% |
| liquid paraffin | 5.65 g | 5.65% |
| isopropyl myristate | 15.00 g | 15.0% |

| aqueous phase | | |
|---|---|---|
| propylene glycol | 20.00 g | 20.0% |
| sodium lauryl sulphate | 0.80 g | 0.8% |
| poloxamer 188 | 1.00 g | 1.0% |
| aq. purif. | 34.8 g | 34.8% |

| active components | | |
|---|---|---|
| penciclovir | 5.00 g | 5.0% |
| hydrocortisone | 1.00 g | 1.0% |

The particle size of the hydrocortisone (Pharmacia & Upjohn micronised USP/EP/BP) was 100% <5 µm, geometric mean diameter 2 µm. The purified water is reverse osmosis treated. The penciclovir was micronised to mean diameter 5 µm.

The oil phase and aqueous phase components are added to respective mixing vessels, which are each heated to 70°C under agitation. When the phases are at an identical temperature, the oil phase is poured onto the aqueous phase from above while continuing to agitate for 3-5 minutes at the highest possible speed which avoids drawing air into the mixture. The thus emulsified mixture is then homogenised and cooled, with continued agitation, to 32-25°C. The active ingredients are added and agitation continued until the active ingredients are wetted and blended in. The mixture is once again homogenised and cooled until the cream thickens, around 30°C, before packaging.

### Comparative Example 1

An acyclovir/hydrocortisone composition employing the prior art MAC carrier described and claimed in EP 44543 was prepared from the following components:

| oil phase | | |
|---|---|---|
| cetostearyl alcohol | 33.75 g | 6.75% |
| vaseline | 50.0 g | 10.0% |
| liquid paraffin | 28.25 g | 5.65% |

| aqueous phase | | |
|---|---|---|
| propylene glycol | 200.0 g | 40.0% |
| sodium lauryl sulphate | 4.0 g | 0.8% |
| poloxamer 188 | 5.0 g | 1.0% |
| aq purif | 148.82 g | 29.8% |

| active components | | |
|---|---|---|
| acyclovir | 28.19 g | 5.0% |
| hydrocortisone | 5.02 g | 1.0% |

The ingredients were blended substantially as described in Example 1.

### Comparative Example 2

An acyclovir/hydrocortisone composition employing a low propylene glycol (PG) concentration (relative to the prior art MAC formulation) but without the isopropyl alkanoic ester (IPM) of the composition of the invention was prepared from the following components:

| oil phase | | |
|---|---|---|
| cetostearyl alcohol | 6.75 g | 6.75% |
| vaseline | 10.00 g | 10.0% |
| liquid paraffin | 5.65 g | 5.65% |

| aqueous phase | | |
|---|---|---|
| propylene glycol | 25.00 g | 25% |
| sodium lauryl sulphate | 0.80 g | 0.8% |
| poloxamer 188 | 1.00 g | 1.0% |
| aq purif | 44.8 g | 44.8% |

| active components | | |
|---|---|---|
| acyclovir | 5.00 g | 5.0% |
| hydrocortisone | 1.00 g | 1.0% |

The ingredients were blended substantially as described in Example 1.

### Biological Example 1

Antiviral efficacy of compositions in accordance with the invention and comparative examples is assayed in a guinea pig model (Alenius et al, J. Inf. Dis. 145 569-573 (1982)). In summary, the model involves establishing a primary herpes simplex virus infection on depilated areas of the dorsal surface of guinea pigs. 24 hours after inoculation, the test and placebo compositions are smeared onto the incipient herpes lesions. This topical administration is repeated twice per day for 3 or 4 days. Lesion appearance is scored daily in a double blinded fashion and the scores from multiple animals averaged, wherein
0 represents no development of lesions,
1 represents a few scattered lesions,
2 represents several lesions, some confluent
3 represents maximum development of confluent lesions

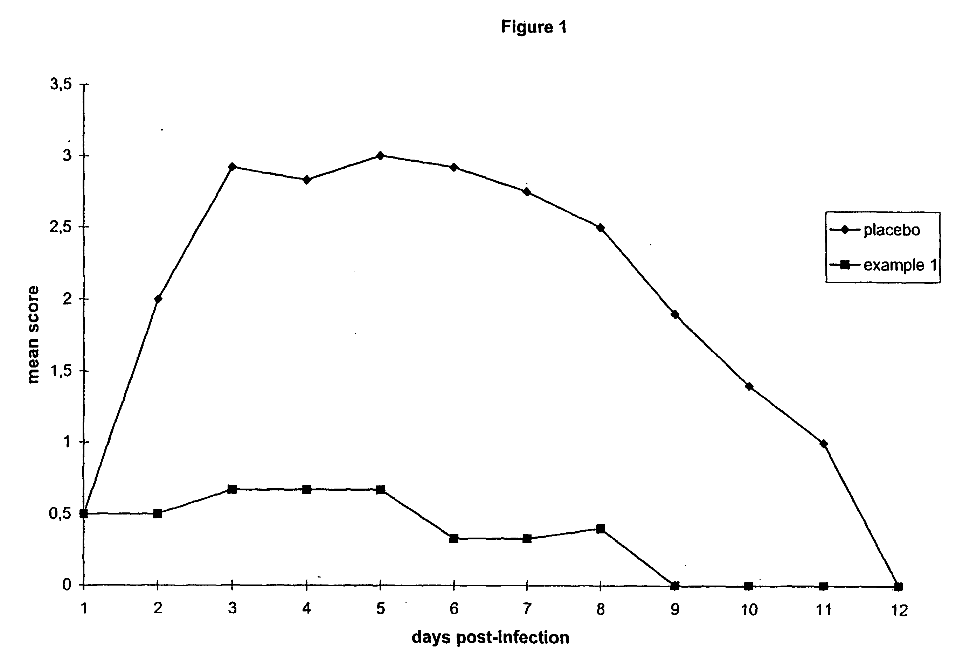

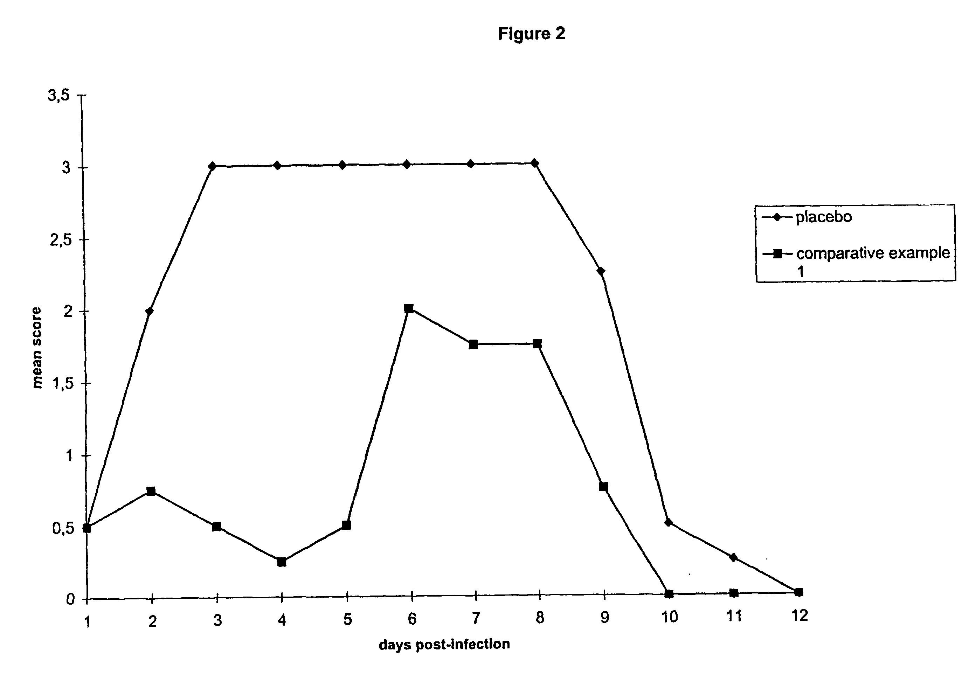

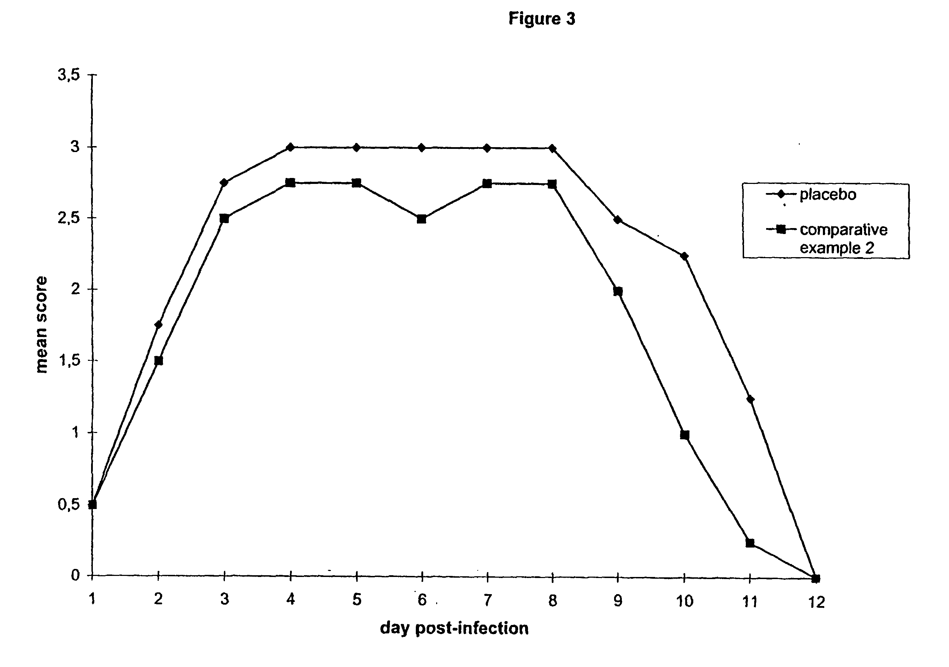

Figure 1 shows the results from a composition in accordance with the invention (Example 1) in comparison to a placebo formulation (the MAC formulation analogous to Comparative Example 1, but without the active ingredients) which represents uninhibited viral growth. Figure 2 shows a substantially prior art composition (Comparative Example 1 - acyclovir & hydrocortisone in a MAC formulation) in comparison to the corresponding placebo. Figure 3 shows a composition falling outside the scope of the invention (Comparative Example 2, low PG/IPM free) in comparison to the placebo.

Referring initially to the placebo formulations it will be apparent that in the absence of the combination of antiviral agent and glucocorticoid, severe lesions form within 3 days and remain unhealed for over 8 days.

A composition embodying the antiviral/glucocorticoid combination broadly in accordance with the abovementioned WO 96/24355 (Comparative Example 1) was freshly produced and comprised an antiviral nucleoside analogue and an antiinflammatory glucocorticoid in a conventional MAC formulation. This formulation displays a retarded development of serious lesions (days 1 to 5) and a somewhat diminished intensity of lesion (maximum score 2 at day 6). As suggested in WO 96/24355 such formulations are thus of utility in the treatment and prophylaxis of herpes infections. However, as described in Assay Example 1, this MAC formulation is not practical in a commercial context for combination regimens.

Turning now to Comparative Example 2 in Fig 3, as would be predicted from the disclosure of the abovementioned EP 44543, WO 91/11187, EP 416 739 and WO 93/00905 directed to various aspects and applications of the MAC formulation, lowering the level of propylene glycol below the "at least 30 weight percent" advised by these patents and applications significantly reduces antiviral efficacy and results in a formulation which is only marginally better than the placebo. In particular, the lesion score is less that one half unit lower than the placebo with barely measurable decreases in time to lesion and time to heal.

In contrast, the compositions of the invention, as depicted in Fig 1, show a significantly improved efficacy relative to both the placebo and the substantially prior art formulation of Fig 2. In particular the lesion score remains at all times less than 1 and is below 0.5 at day 8. Clearly the combination of a reduced propylene glycol concentration in conjunction with the addition of the isopropyl alkanoic acid ester, isporopyl myristate, significantly aids antiviral and therapeutic efficacy of combination products comprising an antiviral and an antiinflammatory glucocorticoid.

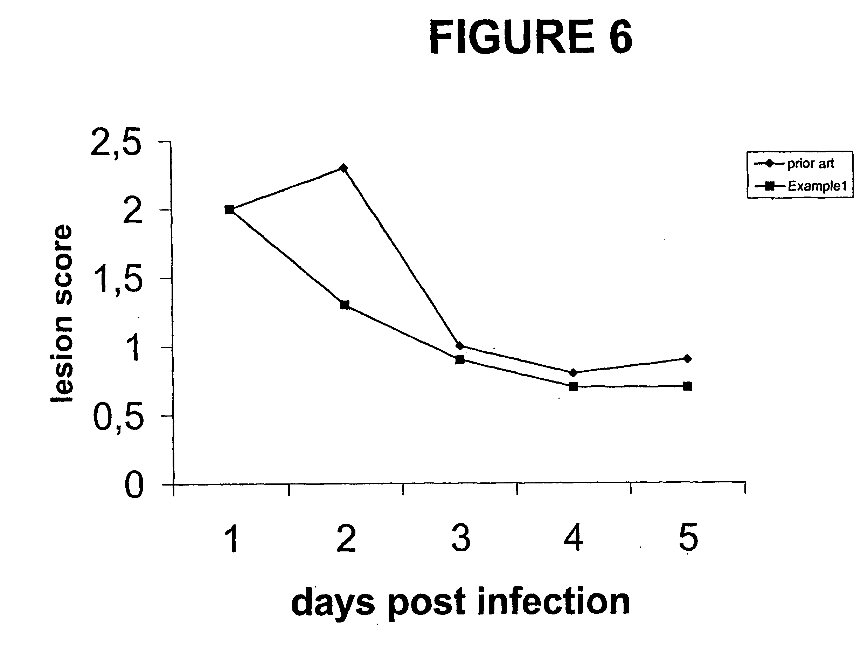

Figure 6 depicts lesion score as a function of days in the guinea pig model broadly as described above but employing a slightly more pathogenic strain of herpes simplex type 1, from an experiment comparing the composition of the invention (Example 1) with a composition also comprising hydrocortisone and acyclovir but prepared according to Example 3 of our copending application WO96/24355. It will be apparent that both compositions provide good healing but the composition of the present invention has an improved performance as regards lesion severity in the early infection. When plotted to lesion disappearance, the area under the curve for the composition of the invention is approximately 10% less than that of the prior art formulation.

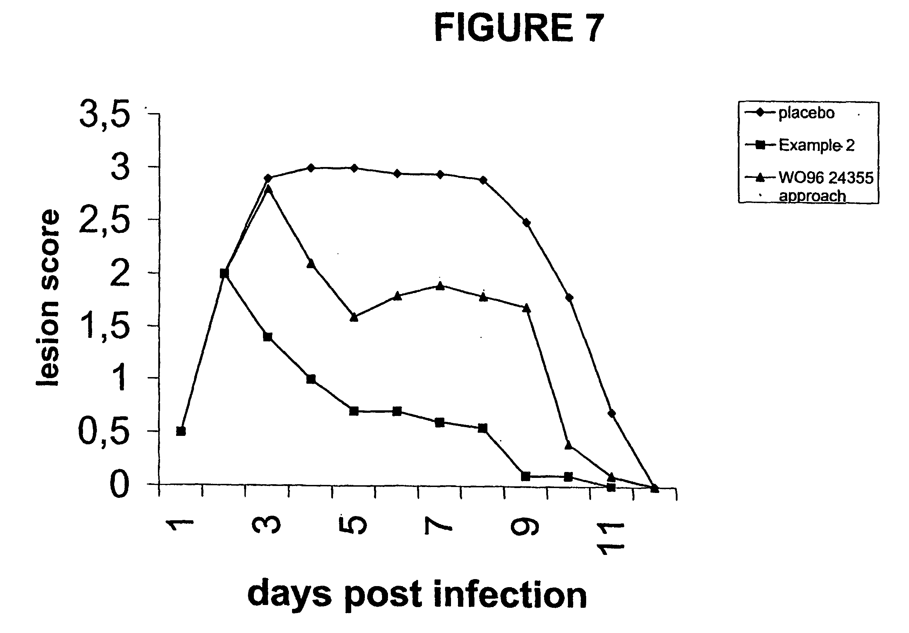

Figure 7 depicts lesion score as a function of days in the guinea pig model described in relation to Fig. 6 from an experiment applying the cream of Example 2 containing an alternative antiviral penciclovir and the glucocorticoid hydrocortisone. For comparison, a cream was prepared analogously to the method described in our copending WO 96/24355 using commercially available Denavir® (penciclovir) (SmithKline Beecham) cream and ACO 1% hydrocortisone. The composition of the invention provides significantly less severe lesions score. The placebo animals treated with the corresponding active ingredient-free carrier are also depicted in Fig 7.

### Assay Example 1

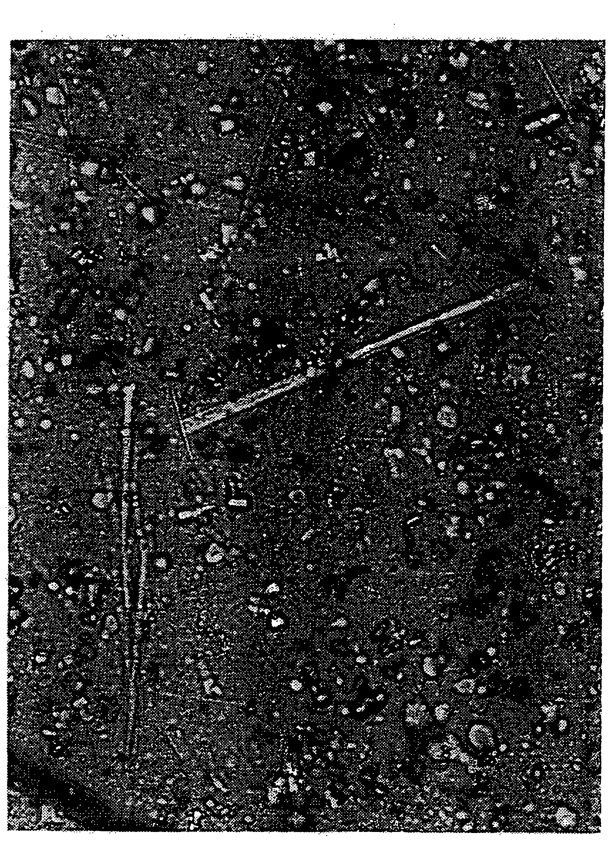

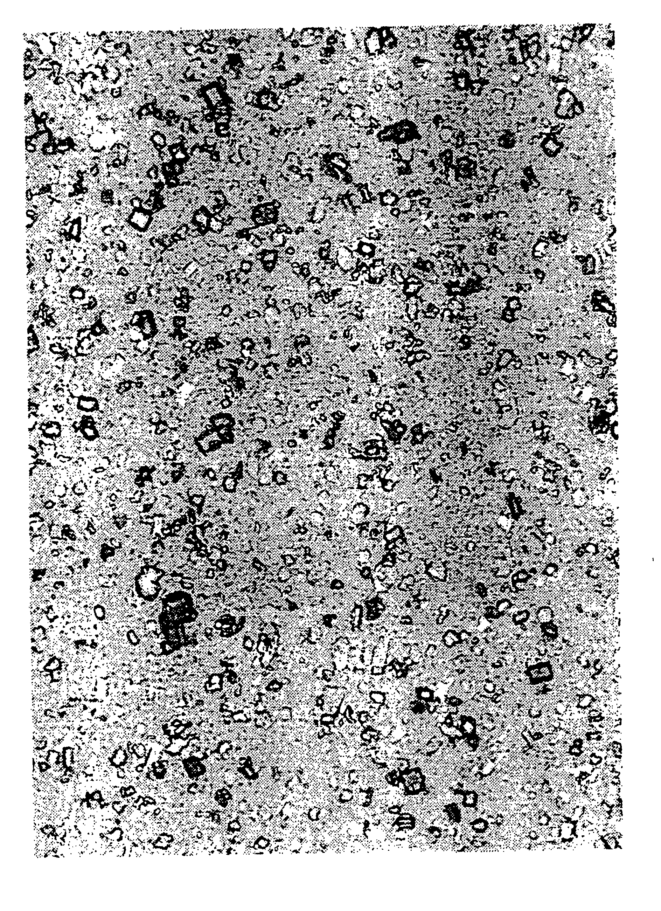

Figures 4 and 5 are photomicrographs at 40x magnification of a composition reflecting a substantially prior art formulation (Figure 4, Comparative Example 1) and a composition of the invention (Figure 5, Example 1).

Referring initially to Fig 5, which depicts a composition of the invention after storage at 25°C for six months, it will be apparent that the crystalline acyclovir and the globules of the oil phase are well dispersed in the aqueous phase. The appearance of this formulation after 6 month's storage is effectively identical to when the formulation was freshly prepared.

In contrast, Fig 4 depicts a 40x magnification of a formulation comprising a glucocorticoid and an nucleoside analogue antiviral agent in a carrier which is formulated in accordance with prior art techniques, namely with around 40 weight % propylene glycol. When freshly prepared, this formulation was microscopically indistinguishable from the corresponding formulation in Fig 5. However, after only 3 months and 3 weeks storage at 25°C, long needle like crystals have grown in this prior art formulation. Analysis shows that these needles comprise the hydrocortisone component of the formulation which has precipitated out of solution in the oil phase, leading to significantly suboptimal topically bioavailable amounts of this component in the formulation. Additionally it will also be apparent that the oil phase is less distinctly dispersed than in the micrograph of Figure 5.

Although the invention has been illustrated with reference to certain proposed and concrete embodiments, exemplified by the antiviral agent acyclovir, the isopropyl alkanoic acid ester IPM etc, it will be appreciated that the invention is not limited by this disclosure and extends to the spirit and scope of the accompanying claims.

## Claims

1. A topical composition in the form of an oil in water emulsion comprising 0.1-10 % (w/w) hydrocortisone and 1-7 % (w/w) of an antiviral nucleoside analogue selected from acyclovir, penciclovir or 9-(4-hydroxy-2-(hydroxymethyl)butyl)guanine in a pharmaceutical carrier comprising propylene glycol and an isopropyl C₁₂-C₂₂ alkanoic acid ester, **characterized in that** the carrier comprises, relative to the total weight of the composition, 15-25 % (w/w) propylene glycol and 10-25 % (w/w) of an isopropyl C₁₂-C₂₂ alkanoic acid ester.

2. The composition according to claim 1, wherein the carrier comprises, relative to the total weight of the composition, 18-22 % (w/w), preferably about 20 % (w/w) propylene glycol.

3. The composition according to claim 1, wherein the carrier comprises, relative to the total weight of the composition, 12-18 % (w/w), preferably about 15 % (w/w) of an isopropyl alkanoic acid ester.

4. The composition according to claim 3, wherein the isopropyl alkanoic acid ester is selected from dodecanate, myristate, palmitate, stearate, eicosanate or behenoate esters, or mixtures thereof.

5. The composition according to claim 4, wherein the isopropyl alkanoic acid ester is isopropyl myristate.

6. The composition according to any of the preceding claims, wherein the nucleoside analogue is acyclovir.

7. The composition according to claim 6, wherein the composition comprises 4-7 % (w/w) acyclovir and 0.5-2 % (w/w) hydrocortisone.

8. The composition according to claim 1, wherein the composition comprises 1-7 % (w/w) penciclovir and 0.5-2 % (w/w) hydrocortisone.

9. Use of a composition as defined in claim 1 in the manufacture of a medicament for the treatment or prophylaxis of herpesvirus infections, wherein the medicament is formulated for topical administration.

## Patentansprüche

1. Topische Zusammensetzung in Form einer Öl-in-Wasser-Emulsion, umfassend 0,1-10% (w/w) Hydrocortison und 1-7% (w/w) eines antiviralen Nukleosidanalogons, ausgewählt aus Acyclovir, Penciclovir oder 9-(4-Hydroxy-2-(hydroxymethyl)butyl)guanin in einem pharmazeutischen Träger, umfassend Propylenglykol und einen Isopropyl-C₁₂-C₂₂-Alkansäureester, **dadurch gekennzeichnet, dass** der Träger im Verhältnis zum Gesamtgewicht der Zusammensetzung 15-25% (w/w) Propylenglykol und 10-25% (w/w) eines Isopropyl-C₁₂-C₂₂-Alkansäureesters umfasst.

2. Zusammensetzung gemäß Anspruch 1, wobei der Träger im Verhältnis zum Gesamtgewicht der Zusammensetzung 18-22% (w/w), vorzugsweise etwa 20% (w/w) Propylenglykol umfasst.

3. Zusammensetzung gemäß Anspruch 1, wobei der Träger im Verhältnis zum Gesamtgewicht der Zusammensetzung 12-18% (w/w), vorzugsweise etwa 15% (w/w) eines Isopropylalkansäureesters umfasst.

4. Zusammensetzung gemäß Anspruch 3, wobei der Isopropylalkansäureester ausgewählt wird aus Dodecanat-, Myristat-, Palmitat-, Stearat-, Eicosanat- oder Behenoat-Estern oder Gemischen davon.

5. Zusammensetzung gemäß Anspruch 4, wobei der Isopropylalkansäureester Isopropylmyristat ist.

6. Zusammensetzung gemäß einem der vorhergehenden Ansprüche, wobei das Nukleosidanalogon Acyclovir ist.

7. Zusammensetzung gemäß Anspruch 6, wobei die Zusammensetzung 4-7% (w/w) Acyclovir und 0,5-2% (w/w) Hydrocortison umfasst.

8. Zusammensetzung gemäß Anspruch 1, wobei die Zusammensetzung 1-7% (w/w) Penciclovir und 0,5-2% (w/w) Hydrocortison umfasst.

9. Verwendung einer Zusammensetzung, wie sie in Anspruch 1 definiert ist, für die Herstellung eines Medikaments für die Behandlung oder Prophylaxe von Herpesvirus-Infektionen, wobei das Medikament für eine topische Verabreichung formuliert ist.

## Revendications

1. Composition topique se présentant sous la forme d'une émulsion huile dans eau comprenant 0,1-10% (w/w) d'hydrocortisone et 1-7% (w/w) d'un analogue de nucléoside antiviral choisi parmi l'acyclovir, le penciclovir ou la 9-(4-hydroxy-2-(hydroxyméthyl)butyl)guanine dans un support pharmaceutique comprenant du propylène glycol et un ester d'isopropyle d'acide alkanoïque en C₁₂-C₂₂, **caractérisé en ce que** le support comprend, relativement au poids total de la composition, 15-25% (w/w) de propylène glycol et 10-25% (w/w) d'un ester d'isopropyle d'acide alkanoïque en C₁₂-C₂₂.

2. Composition selon la revendication 1, dans laquelle le support comprend, relativement au poids total de la composition, 18-22% (w/w), de préférence environ 20% (w/w) de propylène glycol.

3. Composition selon la revendication 1, dans laquelle le support comprend, relativement au poids total de la composition, 12-18% (w/w), de préférence environ 15% (w/w) d'un ester d'isopropyle d'acide alkanoïque.

4. Composition selon la revendication 3, dans laquelle l'ester d'isopropyle d'acide alkanoïque est choisi parmi les esters de dodécanate, de myristate, de palmitate, de stéarate, d'éicosanate ou de behenoate, ou des mélanges de ceux-ci.

5. Composition selon la revendication 4, dans laquelle l'ester d'isopropyle d'acide alkanoïque est le myristate d'isopropyle.

6. Composition selon l'une quelconque des revendications précédentes, dans laquelle l'analogue de nucléoside est l'acyclovir.

7. Composition selon la revendication 6, dans laquelle la composition comprend 4-7% (w/w) d'acyclovir et 0,5-2% (w/w) d'hydrocortisone.

8. Composition selon la revendication 1, dans laquelle la composition comprend 1-7% (w/w) de penciclovir et 0,5-2% (w/w) d'hydrocortisone.

9. Utilisation d'une composition telle que définie dans la revendication 1 pour la préparation d'un médicament pour le traitement ou la prophylaxie d'infections à herpesvirus, dans laquelle le médicament est formulé pour une administration topique.
